# EUROPEAN PATENT SPECIFICATION

(11) **EP 2 236 477 B1**
(45) Date of publication and mention of the grant of the patent: **14.12.2011**
(21) Application number: 09157311.3
(22) Date of filing: 03.04.2009
(51) Int. Cl.: C04B 28/18

(54) **Calcium silicate-based composite cement and methods for the preparation**
Kompositzement auf Calciumsilikatbasis und Verfahren zur Herstellung
Ciment composite à base de silicate de calcium et procédés pour sa préparation

(43) Date of publication of application: 06.10.2010
(73) Proprietor: Ding, Shinn Jyh, Taichung City 408 (TW)
(72) Inventor: Ding, Shinn Jyh, Taichung City 408 (TW)
(74) Representative: 2K Patentanwälte Blasberg Kewitz & Reichel

(56) References cited:
- US-A1- 2008 214 500
- BARDEN, D.: "Silicate performs strongly at bone repair" CHEM. TECHNOL., [Online] vol. 6, no. 3, 31 March 2009 (2009-03-31), page T20, XP002543920 The Royal Society of Chemistry Retrieved from the Internet: URL:http://www.rsc.org/images/CT%20Issue%2 03_tcm18-145465.pdf> [retrieved on 2009-09-01] & DING ET AL: "Novel fast-setting calcium silicate bone cements with high bioactivity and enhanced osteogenesis in vitro" J. MATER. CHEM., vol. 19, 22 January 2009 (2009-01-22), pages 1183-1190, The Royal Society of Chemistry
- DATABASE COMPENDEX [Online] ENGINEERING INFORMATION, INC., NEW YORK, NY, US; LI J ET AL: "Novel calcium silicate/calcium phosphate composites for potential applications as injectable bone cements" XP002543921 Database accession no. E20090411868706 & BIOMEDICAL MATERIALS 20081201 INSTITUTE OF PHYSICS PUBLISHING; DIRAC HOUSE GB, vol. 3, no. 4, 1 December 2008 (2008-12-01),
- DATABASE INSPEC [Online] THE INSTITUTION OF ELECTRICAL ENGINEERS, STEVENAGE, GB; October 2004 (2004-10), HAIYAN LI ET AL: "Preparation and characterization of bioactive and biodegradable wollastonite/poly(D,L-lactic acid) composite scaffolds" XP002543922 Database accession no. 8258171 & Journal of Materials Science: Materials in Medicine Kluwer Academic Publishers USA, vol. 15, no. 10, 2004, pages 1089-1095, ISSN: 0957-4530
- KIYOSHI OKADA ET AL: "Bioactivity of CaSiO3/poly-lactic acid (PLA) composites prepared by various surface loading methods of CaSiO3 powder" JOURNAL OF MATERIALS SCIENCE: MATERIALS IN MEDICINE, KLUWER ACADEMIC PUBLISHERS, BO, vol. 18, no. 5, 11 January 2007 (2007-01-11), pages 899-907, XP019503733 ISSN: 1573-4838
- ILL YONG KIM ET AL: "Preparation of bioactive microspheres of organic modified calcium silicates through sol-gel processing" JOURNAL OF SOL-GEL SCIENCE AND TECHNOLOGY, KLUWER ACADEMIC PUBLISHERS, BO, vol. 45, no. 1, 23 October 2007 (2007-10-23), pages 43-49, XP019577700 ISSN: 1573-4846
- HUGO LEONARDO ROCHA ALVES ET AL: "Injectability evaluation of tricalcium phosphate bone cement" JOURNAL OF MATERIALS SCIENCE: MATERIALS IN MEDICINE, KLUWER ACADEMIC PUBLISHERS, BO, vol. 19, no. 5, 1 December 2007 (2007-12-01), pages 2241-2246, XP019575727 ISSN: 1573-4838
- LEONOR I B ET AL: "Growth of a bonelike apatite on chitosan microparticles after a calcium silicate treatment" ACTA BIOMATERIALIA, ELSEVIER, AMSTERDAM, NL, vol. 4, no. 5, 1 September 2008 (2008-09-01), pages 1349-1359, XP023611098 ISSN: 1742-7061 [retrieved on 2008-03-20]

## Description

### FIELD OF THE INVENTION

This present invention relates to bone cement especially calcium silicate-based bone cements containing polymers and/or oligomers.

### BACKGROUND

Silicon (Si), an important trace element in the early stages of bone formation, increased directly with calcium at relatively low calcium concentrations and then fell below the detection limit at compositions approaching hydroxyapatite (Carlisle EM, Silicon: a possible factor in bone calcification. Science 1970:167:279-280.). The soluble form of silicon may stimulate collagen type I synthesis and osteoblastic differentiation in human osteoblast-like cells (Reffitt DM, Ogston N, Jygdaohsingh R. Orthosilicic acid stimulates collagen type I synthesis and osteoblastic differentiation in human osteoblast-like cells in vitro. Bone 2003;32:127-135.). Silicate-based materials may be promising for use in the reconstruction of frontal sinus and spine, augmentation of craniofacial skeletal defects and osteoporosis, endodontics, and repair of periodontal bone defects. Calcium silicate-based ceramics such as CaSiO₃ (Siriphannon P, Kameshima Y, Yasumori A, Okada K, Hayashi S. Influence of preparation conditions on the microstructure and bioactivity of α-CaSiO3 ceramics: formation of hydroxyapatite in simulated body fluid. J Biomed Mater Res 2000:52:30-39; Sarmento C, Luklinska ZB, Brown L, Anseau M, De Aza PN, De Aza S. In vitro behavior of osteoblastic cells cultured in the presence of pseudowollastonite ceramic. J Biomed Mater Res 2004;69A:351-358.), bioactive glass (Saravanapavan P, Jones JR, Pryce RS, Hench LL. Bioactivity of gel- glass powders in the CaO-SiO2 system: A comparison with ternary (CaO-P2O5-SiO2) and quaternary glasses (SiO2-CaO-P2O5-Na2O). J Biomed Mater Res 2003;66A:110-119.); and mineral trioxide aggregate (Ribeiro DA, Duarte MAH, Matsumoto MA, Marques MEA, Salvadori DMF. Biocompatibility in vitro tests of mineral trioxide aggregate and regular and white Portland cements. J Endod 2005;31:605-607.) were good bioactive materials for bone defect repair in orthopedic and dental surgery because of their excellent bioactivity.

Sol-gel derived calcium silicate materials have been studied for the use of bulk or scaffold. Izquierdo-Barba et al. synthesized a bioactive glass with a composition of 80SiO₂/20CaO (in mol %) by sol-gel method (Izquierdo-Barba 1, Salinas AJ, Vallet-Regi M. In vitro calcium phosphate layer formation on sol-gel glasses of the CaO-SiO2 system. J Biomed Mater Res 1999:47:243-250.). The same research group also found that the rate of apatite formation on the SiO₂-CaO glass with lower SiO₂ (50-70% in mol) is greater than those glasses with higher SiO₂ (80-90% in mol) when soaked in a simulated body fluid (Martinez A, lzquierdo-Barba I, Vallet-Regi M. Bioactivity of a CaO-SiO2 binary glasses system. Chem Mater 2000;12:3080-3088.).

Self-setting cements can be handled by the surgeon in paste form and injected into bone cavities or defects, and set to form a mineral matrix. The setting time is one of most clinically relevant factors. A long setting duration could cause clinical problems due to the cement's inability to maintain shape and support stresses during this time period (Ishikawa K, Miyamoto Y, Takechi M, Toh T, Kon M, Nagayama M, Asaoka K. Non-decay type fast-setting calcium phosphate cement:hydroxyapatite putty containing an increased amount of sodium alginate. J Biomed Mater Res 1997;36:393-399.). Particle size, sintering temperature, liquid phase, and composition of powders, as well as the ratio of liquid to powder, play a crucial role on the setting time of paste materials. On the other hand, the prerequisite for materials to bond to living bone is the formation of a "bone-like" apatite layer, an indicator of bioactivity (the ability to form a chemical bond with living tissue), on their surface in the body. The cement must promote the precipitation of a "bone-like" HA layer on the cement surfaces when exposed to a physiological solution, which may support its ability to integrate with living tissue.

As for the sol-gel derived calcium silicate cements, Chang and his co-workers prepared dicalcium silicate and tricalcium silicate powders using sol-gel methods. With the two calcium silicate powders, they can be mixed with water to produce a calcium silicate cement with the initial setting time of higher than 1 hour, on which the apatite precipitation needed to take several days in simulated body fluid (Zhao W, Wang J, Zhai W, Wang Z, Chang J. The self-setting properties and in vitro bioactivity of tricalcium silicate. Biomaterials 2005;26:6113-6121; Gou Z, Chang J, Zhai W, Wang J. Study on the self-setting property and the in vitro bioactivity of β-Ca2SiO4- J Biomed Mater Res 2005;73B:244-251.). Very recently we proposed preparation of calcium silicate cements consisting of the sol-gel-derived calcium silicate powder as a solid phase and ammonium phosphate solution as a liquid phase. The cements obtained showed not only fast-setting within 9 minutes, as well as highly bioactive, but also enhanced cell proliferation and differentiation (Ding SJ, Shie MY, Wang CY. Novel fast-setting calcium silicate bone cements with high bioactivity and enhanced osteogenesis in vitro. J Mater Chem. DOI: 10.1039/B819033J, 2009, in press). However, it is possible to difficultly deliver to the required site and hard to compact adequately due to relatively poor brittleness resistance of a ceramic-based cement. The polymeric materials such as chitosan, alginate, and gelatin should have the potential to improve the handling properties of calcium-silicate cements because of their inherent elasticity.

Bone and teeth are composite materials consisting mainly of an organic matrix (collagen) and a mineral phase (hydroxyapatite). The successful design of bone substitute materials requires an appreciation of bone structure. Thus, the use of a hybrid composite comprised biopolymer and calcium silicate, resembling the morphology and properties of natural bone, may be one way to solve the problem of ceramics' brittleness without reducing mechanical properties, in addition to possessing good biocompatibility, high bioactivity and great bonding properties.

Li et al. developed a calcium silicate/calcium phosphate composite cement. The liquid phase is a solution of chitosan, sodium phosphate and methylcellulose. (Li J, Qiu ZY, Zhou L, Lin T. Wan Y, Wang SQ, Zhang SM. Novel calcium silicate/calcium phosphate composites for potential applications as injectable bone cements. Biomed. Mater.2008;3:044102.). Li and Chang prepared composite scaffolds of poly(D,L-lactic acid) with bioactive wollastonite (CaSiO₃) by the conventional solvent casting-particulate leaching method. Wollastonite powders were prepared by chemical co-precipitation method (Li H, Chang J. Preparation and characterization of bioactive and biodegradable wollastonite/poly(D,L-lactic acid) composites scaffolds. J Mater Sci: Mater Med 2004;15:1089-1095.). Okada et al. used three sample preparation methods including casting, spin coating, and hot pressing to prepare CaSiO₃/PLA composites. (Okada K, Hasegawa F, Kameshima Y, Nakajima A. Bioactivity of CaSiO3/poly-lactic acid (PLA) composites prepared by various surface loading methods of CaSiO3 powder. J Mater Sci: Mater Med 18:2007:899-907.). Kim et al. prepared microspheres of organic modified calcium silicates using a sol-gel route. (Kim IY. Ohtsuki C, Kawachi G, Kamitakahara M, Cho SB. Preparation of bioactive microphseres of organic modified calcium silicates through sol-gel processing" J Sol-Gel Sci Technol 2008;45:43-49). Leonor et al. treated chitosan microparticles with a calcium silicate solution. (Leonor IB, Baran ET, Kawashita M, Reis RL, Kokubo T, Nakamura T. Growth of a bonelike apatite on chitosan microparticles after a calcium silicate treatment Acta Biomaterialia 2008;4:1349-1359.) Hermansson and Engqvist disclosed an injectable cement consisting of calcium aluminate, zirconium oxide, calcium silicate (in an amount of less than 10 wt%). (Hermansson L, Engqvist H. Injectable cement composition for orthopaedic and dental use. US 2008/0214500)

Chitosan is an abundant, naturally occurring polysaccharide obtained by deacetylation of natural chitin with one of the polysaccharides having a free amino group at the C2 position of the glucose residue of cellulose (Francis Suh JK, Matthew HWT. Application of chitosan-based polysaccharide biomaterials in cartilage tissue engineering: a review. Biomaterials 2000;21:2589-2598.). Due to its numerous desirable properties, e.g., low cost, no antigenicity, chemical inertness, low toxicity, high hydrophilicity, and good film-forming property (Denkbas EB, Odabasi M. Chitosan microspheres and sponges: preparation and characterization. J Appl Polym Sci 2000;76:1637-1643; VandeVord PJ, Matthew HWT, DeSilva SP, Mayton L, Wu B, Wooley PH. Evaluation of the biocompatibility of a chitosan scaffold in mice. J Biomed Mater Res 2002;59:585-590.), chitosan also became a natural choice for biomedical applications such as microspheres, membranes, and scaffolds. Liu and colleagues developed an injectable bone substitute material consisting of chitosan, citric acid and glucose solution as the liquid phase, and tricalcium phosphate powder as the solid phase (Liu H, Li H, Cheng W, Yang Y, Zhu M, Zhou C. Novel injectable calcium phosphate/chitosan composites for bone substitute materials. Acta Biomater 2006;2:557-565.). This material was moldable because of its paste consistency after mixing. Yokoyama et al. developed a chitosan-containing calcium phosphate cement that could be molded into any desired shape due to its chewing-gum-like consistency (Yokoyama A, Yamamoto S, Kawasaki T, Kohgo T, Nakasu M. Development of calcium phosphate cement using chitosan and citric acid for bone substitute materials. Biomaterials 2002-23:1091-1101.). Xu and Simon developed strong and macroporous CPC scaffolds by incorporating chitosan and water-soluble marinitol, and to examine the biocompatibility of the new graft with an osteoblast cell line and an enzymatic assay (Hockin H.K. Xu, Carl G. Simon Jr. Fast setting calcium phosphate-chitosan scaffold: mechanical properties and biocompatibility. Biomaterials 2005; 26:1337-1348.).

Gelatin is a nature polymer, which is obtained from the bovine bone by thermal denaturation or physical and chemical degradation of collagen. Gelatin has been widely employed as a scaffold material in the field of the tissue engineering or the drug carrier because of biocompatibility, biodegradability and nontoxicity (Olsen D, Yang C, Bodo M, Chang R, Leigh S, Baez J, Carmichael D, Perälä M, Hämäläinen ER, Jarvinen M, Polarek J. Recombinant collagen and gelatin for drug delivery. Adv Drug Delivery Rev 2003;55:1547-1567; Tabata Y. Hong L, Miyamoto S, Miyao M, Hashimoto N, lkada Y. Bone formation at a rabbit skull defect by autologous bone marrow cells combined with gelatin microspheres containing TGF-β1. J Biomater Sci Polym Edn 2000;11:891-901.). The advantages of gelatin are the usability with different charges and the easiness of chemical modification. Fujishiro and coworkers found that addition of gelatin gel to α- tricalcium phosphate cement resulted in the formation of a porous solid possessing pores of 20-100 µm in diameter whose pore diameter increased with increasing gelatin gel content (Fujishiro Y, Takahashi K, Sato T. Preparation and compressive strength of α-tricalcium phosphate/gelatin gel composite cement. J Biomed Mater Res 2001; 54:525-530.). The compressive strength of α- tricalcium phosphate cement after 1 week increased from 9.0 to 14.1 MPa with increasing gelatin gel content up to 5 wt% and thereafter decreased. The use of chitosan fiber and gelatin is to reinforce the mechanical property of CPC (Pan Z, Jiang P, Fan Q, Ma B. Cai H. Mechanical and biocompatible influences of chitosan fiber and gelatin on calcium phosphate cement. J Biomed Mater Res 2007;82B: 246-252). Gelatin at the mass fraction of 5% and chitosan fiber at the volume fraction of 30% are optimal for this reinforcement mode.

Barden, D. "Silicate performs strongly at bone repair", Chem. Technol. (online) vol. 6, no. 3, 31 March 2009, page T20, The Royal Society of Chemistry discloses a calcium silicate bone cement to which gelatin and chitosan are added in order to improve the injectability. A step of adding the gelatin/chitosan prior to grinding is not disclosed.

### SUMMARY OF THE INVENTION

It is an object of the present invention to provide a method for producing an improved calcium silicate-based bone cement.

Another object of the present invention is to provide an improved composition for polymer-containing calcium silicate bone cement.

The above objects are achieved by a method for producing polymer-containing calcium silicate bone cement according to claim 1 and by a composition for polymer-containing calcium silicate bone cement according to claim 9. Further advantageous embodiments are the subject-matter of the dependent claims.

### BRIEF DESCRIPTION OF THE DRAWINGS

The following detailed description, given by way of examples and not intended to limit the invention to the embodiments described herein, will best be understood in conjunction with the accompanying drawings, in which:
Figure 1 illustrates XRD patterns for (A) S60C40, (B) S50C50, (C) S40C60, and (D) S30C70 powders after sintering at different temperature.
Figure 2 illustrates XRD patterns for the five cements which powders were prepared by sintering at 800°C and the liquid phase was water.
Figure 3 illustrates XRD patterns for the five calcium silicate-based cements containing (A) gelatin or (B) chitosan.
Figure 4 illustrates DTS values of the five calcium silicate-based cements containing different amounts of gelatin (GLT). Water and 5 wt% chitosan (CTS) solution were used as the liquid phase to prepare the cement specimens.
Figure 5 illustrates setting time values of the five calcium silicate-based cements containing with and without 5 wt% gelatin (GLT). Water and 5 wt% chitosan (CTS) solution were used as the liquid phase to prepare the cement specimens.
Figure 6 illustrates the injectability of the S50C50 cements with and without 5 wt% gelatin (GLT). Water and 5 wt% chitosan (CTS) solution were used as the liquid phase to prepare the cement specimens.
Figure 7 illustrates DTS values of (A) S60C40, (B) S50C50, (C) S40C60, and (D) S30C70 cement specimens with and without 5 wt% gelatin (GLT) before and after immersion in simulated body fluid for predetermined periods of time. Water and 5 wt% chitosan (CTS) solution were used as the liquid phase to prepare the cement specimens.
Figure 8 illustrates surface SEM micrographs of (A) S60C40, (B) S50C50, (C) S40C60, and (D) S30C70 cement specimens after immersion for 1 day in simulated body fluid. Water was used as a liquid phase to prepare the cement specimens.
Figure 9 illustrates surface SEM micrographs of (A) hardened S50C50 cement specimens containing (B) gelatin, (C) chitosan, and (D) gelatin and chitosan after immersion for 1 hour in simulated body fluid. Water and 5 wt% chitosan solution were used as the liquid phase to prepare the cement specimens. The arrows indicate the apatite precipitates.
Figure 10 illustrates WST-1 assay for attachment and proliferation of human mesenchymal stem cells cultured on the S50C50 cements with and without 5 wt% gelatin (GLT) at various incubation periods. Water and 5 wt% chitosan (CTS) solution were used as the liquid phase to prepare the cement specimens.
Figure 11 illustrates SEM micrographs of human mesenchymal stem cells cultured on (A) the S50C50 cements containing (B) gelatin, (C) chitosan, and (D) gelatin and chitosan for 1 day. Water and 5 wt% chitosan solution were used as the liquid phase to prepare the cement specimens.

### DETAILED DESCRIPTION OF THE INVENTION

The present invention provides a method for producing calcium silicate-based bone cement, comprising the following steps:
from the group consisting of calcium oxide, calcium hydroxide, calcium acetate, calcium carbonate and calcium chloride, and wherein the silicon compound is selected from the group consisting of silicon dioxide and silicon tetraacetate;
   (b) treating the mixture of step (a) with a sol-gel process;
   (c) heating the mixture of step (b) ;
   (d) adding gelatin to the mixture of step (c) in the range of 2-30 total wt% for enhancing plasticity;
   (e) grinding the mixture of step (d) into powder; and
   (f) adding the powder to water or phosphate.

According to the present invention the material for enhancing plasticity is gelatin weighed in the range of 2-30 wt%.

In a preferred embodiment, said mixture has a molar ratio of calcium to silicon which ranges from 10 to 0.1. In a more preferred embodiment, the mixture has the molar ratio of calcium to silicon range from 4 to 0.25.

The sol-gel process of said method comprises the following steps:
(a) mixing the mixture calcium and silicon precursors with a diluted solvent selected from the group consisting of ethanol and nitric acid for 1~12 hours;
(b) placing the mixture at the temperature of 20 ∼ 100°C for 1~7 days; and
(c) drying the mixture at -40 ~150°C.

The heating process of said method comprises the following steps:
(a) heating the dried mixture to 700~1300°C at the rate of 1~40 °C/min;
(b) maintaining the mixture at a constant temperature ranges from 700~1300°C; and
(c) cooling the mixture to room temperature by air-cooling, water-cooling or fast cooling techniques to obtain calcium silicate powder.

The adding process comprises the following steps:
(a) adding the gelatin to the calcium silicate powder; and
(b) mixing the powder using a conditioning mixer for 5 ~ 30 min.

The grinding process comprises the following steps:
(a) mixing the calcium silicate powder containing the materials for enhancing plasticity with alcohol;
(b) grinding the powder with a ball miller for 0.5 ~ 3 days; and
(c) drying the powder at -40~60°C.

In a preferred embodiment, the particle size of the powder ranges from 0.01 to 50 micrometers;

In a preferred embodiment, the powder is added to water for 10 ∼ 60 seconds and the powder to water is at a ratio of 1 g/0.3-2 mL. In a more preferred embodiment, the powder to water is at a ratio of 1 g/0.4-0.8 mL.

In a preferred embodiment, the powder is added to phosphate solution for 10 ∼ 60 seconds and the powder to phosphate solution is at a ratio of 1 g/0.3-2 mL. In the best embodiment, the powder to phosphate solution is at a ratio of 1 g/0.4-0.8 mL.

In a preferred embodiment, the phosphate solution contains an anion selected from phosphate radical (PO₄³⁻), monohydric phosphate radical (HPO₄²⁻) or dihydric phosphate radical (H₂PO₄⁻) with a concentration of 0.12∼5 M, and the phosphate solution contains a cation selected from ammonium, or a member of group I A.

Preferably, the cation of the phosphate solution is ammonium, sodium or potassium.

The present invention further provides a composition for polymer-containing calcium silicate bone cement, comprising
(a) calcium salt selected from the group consisting of calcium oxide, calcium hydroxide, calcium acetate, calcium carbonate and calcium chloride;
(b) silicon compound selected from the group consisting of silicon dioxide and silicon tetraacetate;
(c) gelatin in the range of 2-30 total wt%; and
(d) pharmacological acceptable.

The pharmacological acceptable solution is water, sodium chloride solution, calcium chloride solution or phosphate solution.

Furthermore, the composition can be applied to orthopedic surgery, spine fusion surgery or dental application. It can also serve as replacement bone or tooth material. When applied to human, it can further comprise an excipient for oral administration.

### EXAMPLE

The example below is non-limiting and is merely representative of various aspects and features of the present invention.

Comparative Example 1 to improve an understanding of the invention

### Phase composition of calcium silicate powders

Tetraethyl orthosilicate (Si(OC₂H₅)₄, TEOS) and calcium nitrate (Ca(NO₃)₂·4H₂O) were used as precursors for SiO₂ and CaO, respectively, and nitric acid as catalyst. Ethanol was used as the solvent. The molar ratio of SiO₂/CaO is in the range of 7/3 to 3/7, as listed in Table 1. Various calcium silicate powders were synthesized by sol-gel method. The general procedure of sol-gel route, such as hydrolysis and ageing, was adopted. Briefly, TEOS was hydrolyzed with the sequential addition of 2 N HNO₃ and absolute ethanol for 1 hour stirring separately. The required amount of Ca(NO₃)₂·4H₂O was added to the above ethanol solution, the mixed solutions were stirred for an another 1 hour. The molar ratio of (HNO₃+H₂O):TEOS:ethanol was 10:1:10. The sol solution was sealed and aged at 60°C for 1 day.

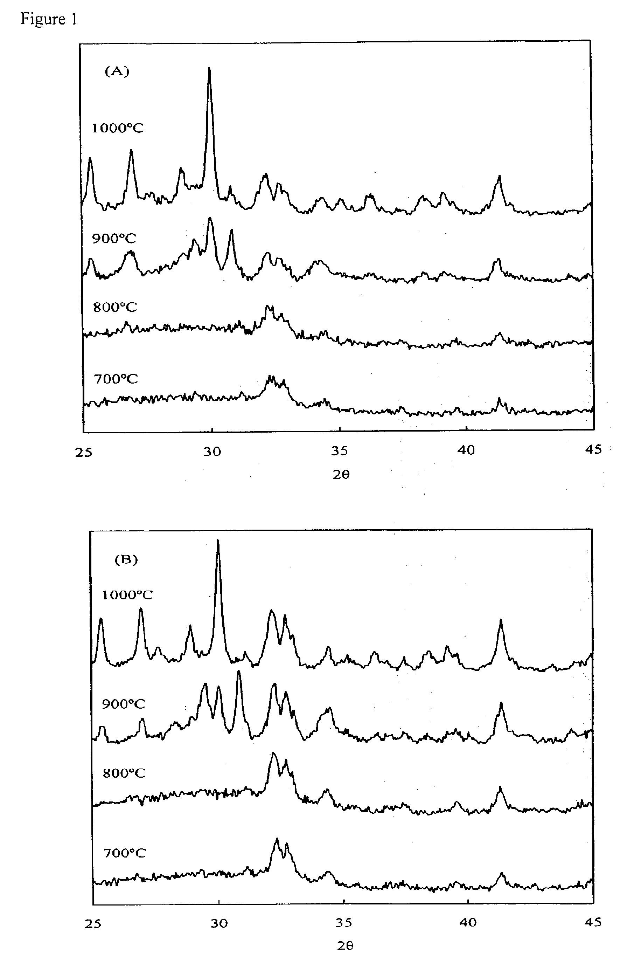

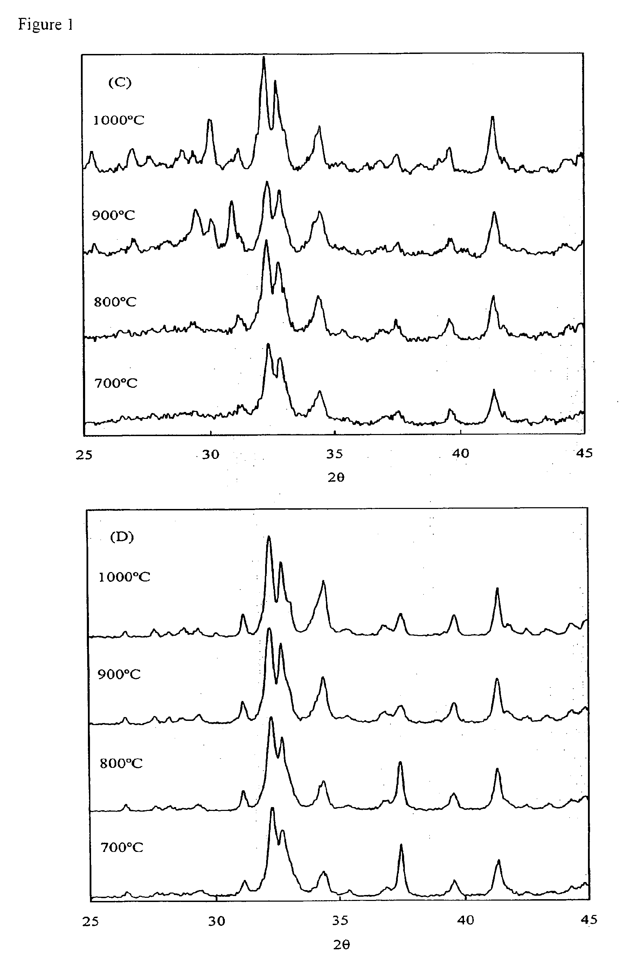

After solvent vaporization of the above-mentioned mixture solution in an oven at 120°C, the as-dried gel was heated in air to 700, 800, 900, or 1000°C for holding 2 hours, and then cooled to room temperature to produce various calcium silicate powders. Phase analysis was performed using Shimadzu XD-D1 X-ray diffractometer (XRD) with Ni-filtered Cukα radiation operated at 30 kV and 30 mA at a scanning speed of 1°/min. XRD spectra of various as-prepared powders at different sintering temperature are presented in Fig. 1. The diffraction maximum between 29 and 35° at 2θ can be attributed to different crystalline phases of calcium silicates, such as wollastonite and dicalcium silicate. The crystallinity of the powders increased with the increasing sintering temperature.

**Table 1 Composition (molar ratio), setting time and diametral tensile strength (DTS) of the five SiO₂-CaO cements prepared by using powders at different sintering temperature after mixing with water or 0.5 M Na₂HPO₄**

| Sample Code | Composition | Sintering temperature (°C) | Water | | 0.5 M Na₂HPO₄ | |
|---|---|---|---|---|---|---|
| | SiO₂:CaO | | Setting time (min) | DTS (MPa) | Setting time (min) | DTS (MPa) |
| S70C30 | 7:3 | 700 | 38.4 ± 1.9 | 0.1 ± 0.0 | 35.4 ±1.7 | 0.2 ± 0.0 |
| | | 800 | 42.6 ± 2.1 | 0.6 ± 0.1 | 36.4 ± 2.5 | 0.3 ± 0.1 |
| | | 900 | 44.1 ± 3.0 | 0.4 ± 0.1 | 38.5 ± 1.6 | 0.4 ± 0.1 |
| | | 1000 | 46.0 ± 2.6 | 0.3 ± 0.1 | 41.0 ± 2.1 | 0.5 ± 0.1 |
| S60C40 | 6:4 | 700 | 33.5 ± 1.6 | 0.2 ± 0.0 | 32.8 ± 1.7 | 0.2 ± 0.0 |
| | | 800 | 33.9 ± 2.3 | 2.2 ± 0.3 | 31.5 ± 2.6 | 2.0 ± 0.3 |
| | | 900 | 34.1 ± 1.5 | 1.6 ± 0.3 | 31.9 ± 1.5 | 2.4 ± 0.3 |
| | | 1000 | 35.6 ± 2.1 | 2.3 ± 0.3 | 33.3 ± 2.2 | 2.7 ± 0.4 |
| S50C50 | 5:5 | 700 | 24.8 ± 1.5 | 1.8 ± 0.3 | 24.0 ± 1.9 | 1.7 ± 0.3 |
| | | 800 | 28.3 ± 2.6 | 2.8 ± 0.4 | 25.6 ± 1.4 | 2.8 ± 0.4 |
| | | 900 | 28.5 ± 2.1 | 2.4 ± 0.2 | 27.4 ± 1.7 | 2.8 ± 0.3 |
| | | 1000 | 31.1 ± 1.8 | 2.1 ± 0.2 | 30.4 ± 1.6 | 3.2 ± 0.5 |
| S40C60 | 4:6 | 700 | 15.3 ± 1.7 | 1.8 ± 0.3 | 14.9 ± 1.1 | 1.7 ± 0.2 |
| | | 800 | 16.8 ± 2.2 | 2.2 ± 0.4 | 13.1 ± 1.9 | 2.1 ± 0.4 |
| | | 900 | 16.0 ± 2.1 | 1.8 ± 0.3 | 15.5 ± 1.9 | 2.9 ± 0.6 |
| | | 1000 | 17.4 ± 1.6 | 2.0 ± 0.2 | 17.5 ± 1.6 | 1.6 ± 0.2 |
| S30C70 | 3:7 | 700 | 10.3 ± 1.7 | 1.6 ± 0.2 | 9.3 ± 1.0 | 1.4 ± 0.2 |
| | | 800 | 11.6 ± 1.7 | 1.1 ± 0.2 | 10.6 ± 1.7 | 1.3 ± 0.3 |
| | | 900 | 11.3 ± 1.7 | 1.1 ± 0.3 | 9.9 ± 1.2 | 0.9 ± 0.1 |
| | | 1000 | 12.3 ± 1.8 | 1.1 ± 0.2 | 10.4 ± 1.7 | 1.2 ± 0.2 |

### Comparative Example 2 to improve an understanding of the invention

### Effect of sintering temperature on setting time and diametral tensile strength of the cements

Tetraethyl orthosilicate (Si(OC₂H₅)₄, TEOS) and calcium nitrate (Ca(NO₃)₂·4H₂O) were used as precursors for SiO₂ and CaO, respectively, and nitric acid as catalyst. Ethanol was used as the solvent. The S70C30, S60C40, S50C50, S40C60, and S30C70 powders were synthesized by sol-gel method and sintered at different temperatures of 800, 900, or 1000°C for 2 hour. After which, the as-sintered powders were ball-milled using agate jars with agate grinding media in an ethanol medium for 12 hours under Retsch centrifugal ball mill S 100. After drying, in order to prepare the cement, 0.2 g powder was mixed with 0.1 mL of water or 0.5 M Na₂HPO₄. The setting time of the cements was tested using the 400-g Gillmore needle with a diameter of 1 mm according to the international standard ISO 9917-1 for water-based cements (ISO 9917-1, Dentistry-water-based cements part1: powder/liquid acid-base cements. International Standard Organization, 2003). The setting time was recorded when the needle failed to create an indentation of 1 mm in depth in three separate areas. After mixing the cement specimens were placed into a cylindrical stainless steel mould to form the specimen dimension of 6 mm (diameter) × 3 mm (height), and stored in an incubator at 100% relative humidity and 37°C for 1 day. Eight specimens from each group were tested. The diametral tensile testing of cement specimens was conducted on an EZ-Test machine (Shimadzu, Kyoto, Japan) at a loading rate of 0.5 mm/min. The diametral tensile strength (DTS) value of the cement specimens was calculated from the relationship DTS = 2P/πbw, where P is the peak load (Newton), b is the diameter (mm) and w is the thickness (mm) of the specimen. The maximal compression load at failure was obtained from the recorded load-deflection curves. At least twenty specimens were used each group. Table 1 presents the results of setting times and DTS values of five calcium silicate cement specimens. When mixed with water, the setting time ranged from 43 to 11 minutes, significantly depending on the used calcium silicate powders. With the increase in the concentration of calcium component, the setting time of the cement became shorter. The cement specimens containing SiO₂/CaO molar ratio ranging from 6:4 to 4:6 had a higher strength than the other two cement specimens. The sintering temperature used for preparation of solid phases did not distinctly affect the setting time and DTS of the cement specimens. On the other hand, 0.5 M Na₂HPO₄ as a liquid phase presented a similar trend to water.

### Comparative Example 3 to improve an understanding of the invention

### Phase composition of the cement specimens after mixing with water

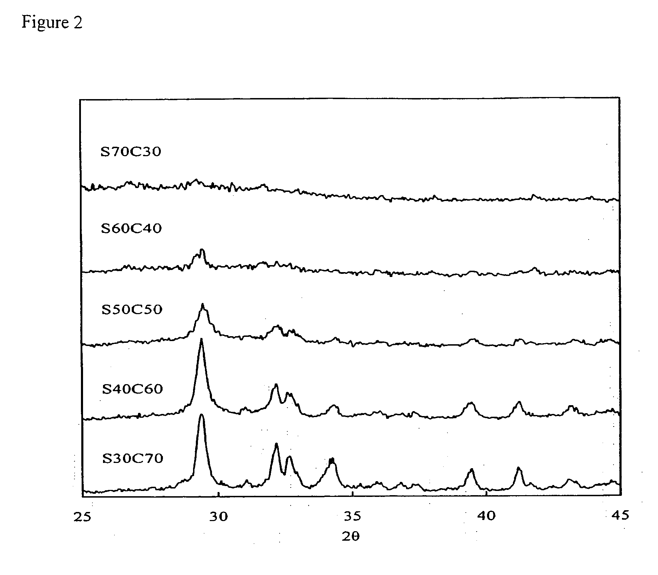

Tetraethyl orthosilicate (Si(OC₂H₅)₄, TEOS) and calcium nitrate (Ca(NO₃)₂·4H₂O) were used as precursors for SiO₂ and CaO, respectively, and nitric acid as catalyst. Ethanol was used as the solvent. The S70C30, S60C40, S50C50, S40C60, and S30C70 powders were synthesized by sol-gel method and sintered at 800°C for 2 hour, and then were ball-milled using agate jars with agate grinding media in an ethanol medium for 12 hours under Retsch centrifugal ball mill S 100. After drying, in order to prepare the cement, 0.4 g powder was mixed with 0.2 mL of water. The cement was stored in an incubator at 100% relative humidity and 37°C for 1 day. X-ray diffractometer (XRD) was used to analyze the phase composition of the hardened cement specimens. The products of the hydration process are calcium silicate hydrates (CaO-SiO₂-H₂O, C-S-H) at 29.3° as well as incompletely reacted inorganic component phases as shown in Fig. 2.

### Comparative Example 4 to improve an understanding of the invention

### Effect of precursors

Different precursors for SiO₂ and CaO, as listed in Table 2, are used. The precursors for silicon included Tetraethyl orthosilicate (Si(OC₂H₅)₄, TEOS), silicon dioxide (SiO₂), and silicon tetraacetate (Si(CH₃COO)₄). Calcium precursors were calcium nitrate (Ca(NO₃)₂·4H₂O), calcium oxide (CaO), calcium hydroxide (Ca(OH)₂), calcium acetate (Ca(CH₃COO)₂), and calcium chloride (CaCl₂). Nitric acid and ethanol were used as the catalyst and solvent, respectively. Calcium silicate powders with equimolar Si/Ca ratio (S50C50) were synthesized by sol-gel method. The general procedure of sol-gel route, such as hydrolysis and ageing, was adopted. After ageing at 60°C and solvent vaporization in an oven at 120°C, the as-dried gel was heated in air to 900°C for holding 2 hour, and then cooled to room temperature to produce various calcium silicate powders. The sintered powders were further ball-milled using agate jars with agate grinding media in an ethanol medium for 12 hours under Retsch centrifugal ball mill S 100. After drying, in order to prepare the cement, the powder was mixed with the (NH₄)₂HPO₄-NH₄H₂PO₄ buffer solution (pH 7.4). The setting time of the cements was tested using the 400-g Gillmore needle with a diameter of 1 mm according to the international standard ISO 9917-1 for water-based cements. In addition, the cement specimens were placed into a cylindrical stainless steel mould to form the specimen dimension of 6 mm (diameter) × 3 mm (height), and stored in an incubator at 100% relative humidity and 37°C for 1 day. The diametral tensile testing of cement specimens was conducted on an EZ-Test machine (Shimadzu, Kyoto, Japan) at a loading rate of 0.5 mm/min. At least ten specimens from each group were tested. Table 2 presents the results of setting times and DTS values of cement specimens. The setting time and DTS values appreciably depended on the type of precursors.

**Table 2 Setting time and diametral tensile strength (DTS) of the S50C50 cements prepared by mixing with ammonium hydrogen solution**

| | TEOS | | SiO₂ | | Si(CH₃COO)₄ | |
|---|---|---|---|---|---|---|
| | Setting time (min) | DTS (MPa) | Setting time (min) | DTS (MPa) | Setting time (min) | DTS (MPa) |
| Ca(NO₃)₂ | 3.7 ± 0.8 | 2.3 ± 0.1 | 12.3 ± 0.8 | 0.4 ± 0.0 | 6.0 ± 1.3 | 2.5 ± 0.1 |
| CaO | 15.2 ± 1.2 | 0.5 ± 0.0 | 20.0 ± 2.7 | 1.7 ± 0.0 | 15.7 ± 2.0 | 1.0 ± 0.0 |
| CaCl₂ | 10.3 ± 1.5 | 0.5 ± 0.1 | 15.5 ± 1.0 | 1.2 ± 0.1 | 12.5 ± 1.4 | 0.7 ± 0.1 |
| Ca(OH)₂ | 32.5 ± 1.9 | 1.9 ± 0.1 | 38.8 ± 2.8 | 0.8 ± 0.1 | 27.2 ± 1.7 | 2.0 ± 0.1 |
| Ca(CH₃COO)₂ | 7.8 ± 0.8 | 1.8 ± 0.1 | 12.5 ± 1.4 | 0.7 ± 0.0 | 13.8 ± 1.0 | 1.8 ± 0.1 |

### Example 5

### Phase composition of the cement specimens containing gelatin and chitosan

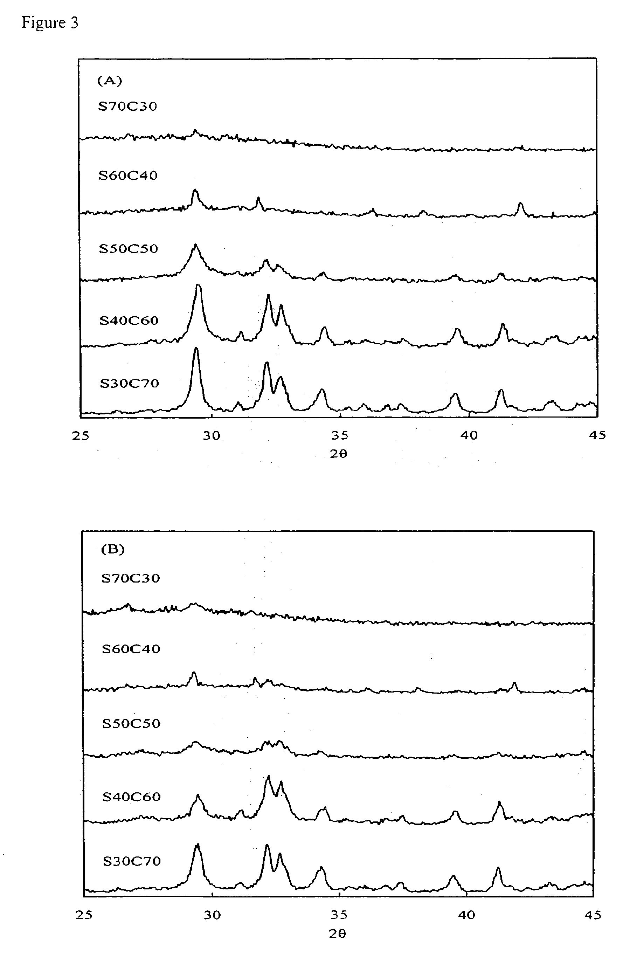

The sol-gel-derived S70C30, S60C40, S50C50, S40C60, and S30C70 powders were sintered at 800°C for 2 hours. 5 wt% of gelatin was added to the sintered powders. Then, the mixtures with and without gelatin were ball-milled using agate jars with agate grinding media in an ethanol medium for 12 hours under Retsch centrifugal ball mill S 100. After drying, in order to prepare the cement, 0.2 g powder was mixed with 0.1 mL of water or 5 wt% of chitosan solution. Chitosan oligosaccharide lactate powder was dissolved in distilled water at chitosan/(chitosan + water) = 5% mass fraction to form the chitosan solution. After mixing, the specimens were stored in an incubator at 100% relative humidity and 37°C for I day. XRD was used to analyze the phase composition of the hardened cement specimens. The product of the hydration process was a calcium silicate hydrate (CaO-SiO₂-H₂O, C-S-H) gel, as shown in Fig. 3. The presence of chitosan in the cement specimen reduced the peak intensity of C-S-H (Fig. 3B).

### Example 6

### Effect of gelatin and chitosan contents on diametral tensile strength and setting time of the cements

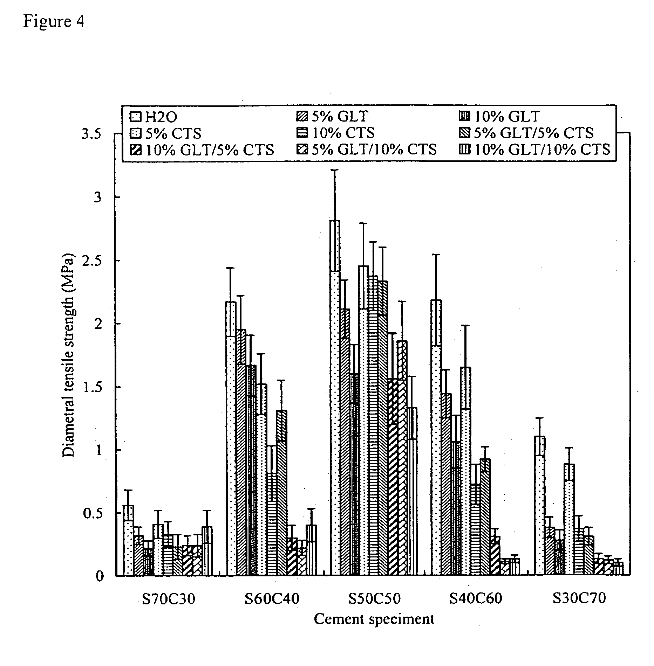

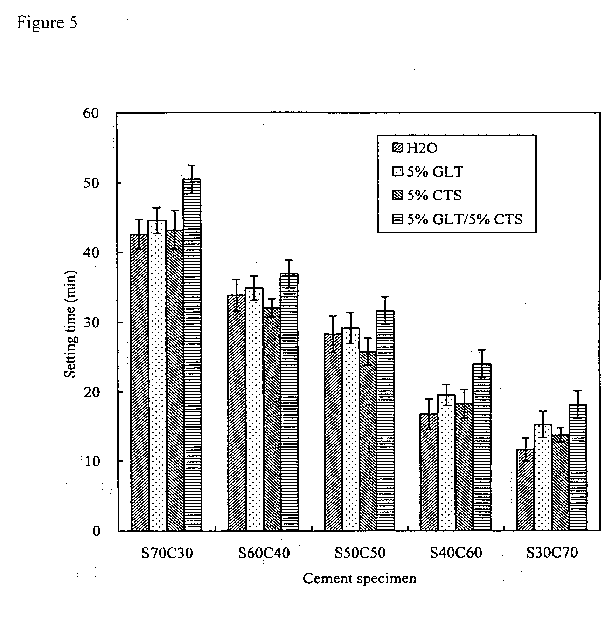

The sol-gel-derived S70C30, S60C40, S50C50, S40C60, and S30C70 powders were sintered at 800°C for 2 hours. 5 and 10 wt% of gelatin (GLT) was added to the sintered powders. Then, the mixtures with and without gelatin were ball-milled using agate jars with agate grinding media in an ethanol medium for 12 hours under Retsch centrifugal ball mill S 100. After drying, in order to prepare the cement, 0.2 g powder was mixed with 0.1 mL of water or chitosan (CTS) solution. Chitosan oligosaccharide lactate powder was dissolved in distilled water at chitosan/(chitosan + water) = 5 or 10% mass fraction to form the chitosan solution. After mixing, the specimens were stored in an incubator at 100% relative humidity and 37°C for 1 day. The setting time of the cements was tested using the 400-g Gillmore needle with a diameter of 1 mm according to the international standard ISO 9917-1 for water-based cements. The setting time was recorded when the needle failed to create an indentation of 1 mm in depth in three separate areas. In addition, the cement specimens were placed into a cylindrical stainless steel mould to form the specimen dimension of 6 mm (diameter) × 3 mm (height), and stored in an incubator at 100% relative humidity and 37°C for 1 day. Eight specimens from each group were tested. The diametral tensile testing of cement specimens was conducted on an EZ-Test machine (Shimadzu, Kyoto, Japan) at a loading rate of 0.5 mm/min. At least twenty specimens were used each group. Figure 4 shows diametral tensile strength (DTS) of various cement specimens. The addition of gelatin or chitosan could decrease DTS of the cement specimens. Similarly, they might also affect the setting time as shown in Figure 5.

### Example 7

### Injectability of the cement

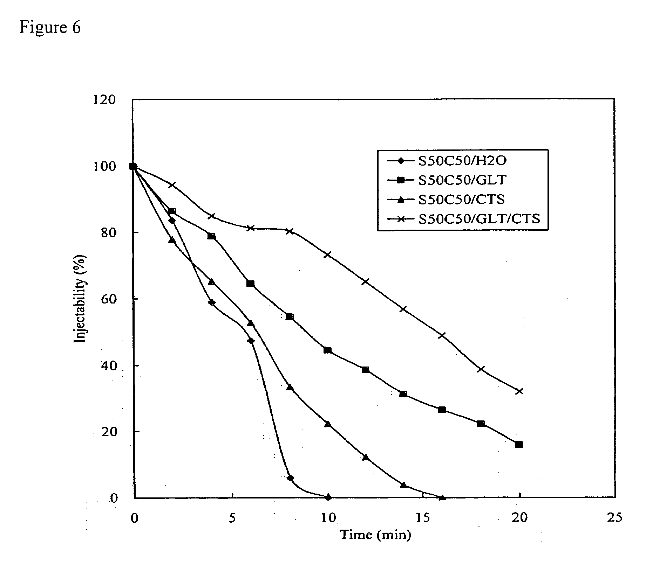

The sol-gel-derived S50C50 powders were sintered at 800°C for 2 hours. 5 wt% of gelatin (GLT) was added to the sintered powders. Then, the mixtures with and without gelatin were ball-milled using agate jars with agate grinding media in an ethanol medium for 12 hours under Retsch centrifugal ball mill S 100. After drying, in order to prepare the cement, the powder was mixed with water or 5 wt% of chitosan (CTS) solution liquid-to-powder (L/P) ratio of 0.6 mL/g. The injectability was evaluated by extruding the paste through a disposable syringe of 5-mL equipped with the needle having an opening of 2.0 mm. The appropriate amount of cement paste was used, and "injectability" was taken to mean the percentage by weight of that part of this amount of paste, which could be extruded from such syringes by hand. The injectability of the cement pastes was determined 2 min after the beginning of mixing powder and liquid. Figure 6 shows the injectability of the S50C50 cement with and without gelatin and chitosan. Both gelatin and chitosan can improve the injectabilty of the cement specimens.

### Example 8

### Diametral tensile strength and morphology for various cement specimens after immersion in simulated body fluid

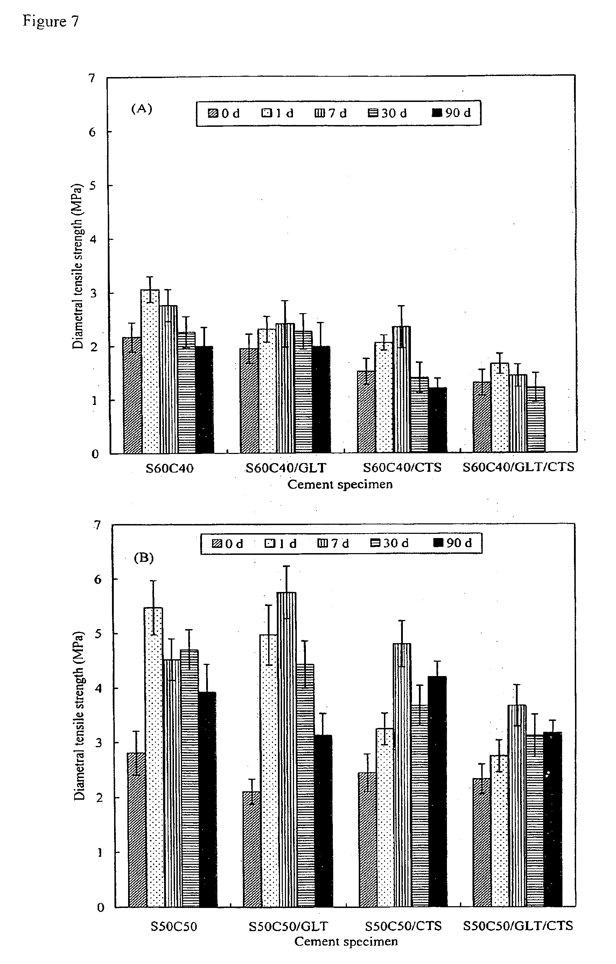

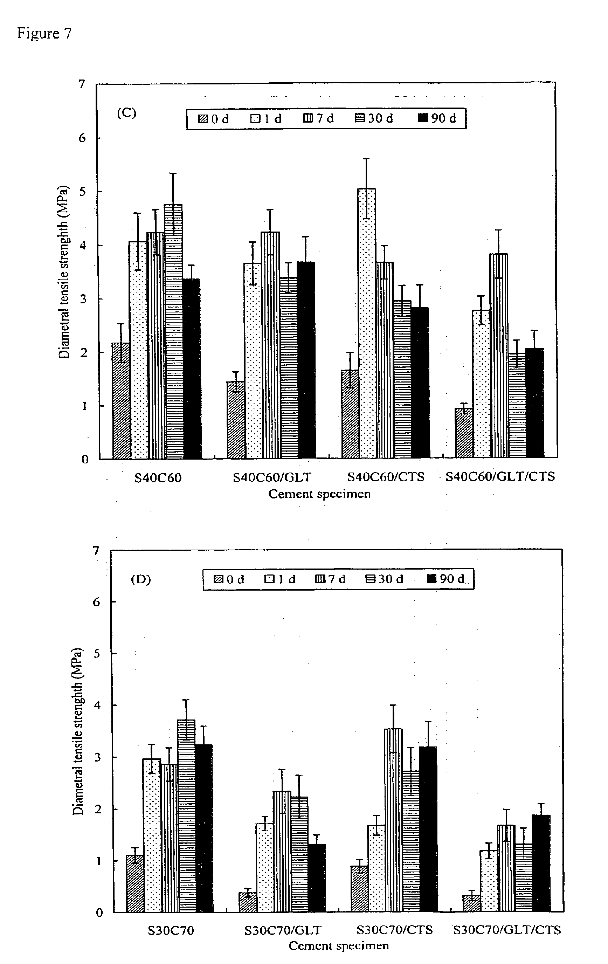

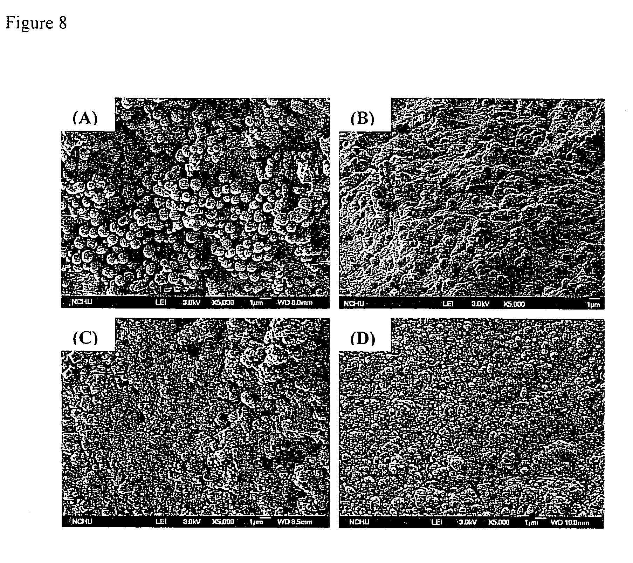

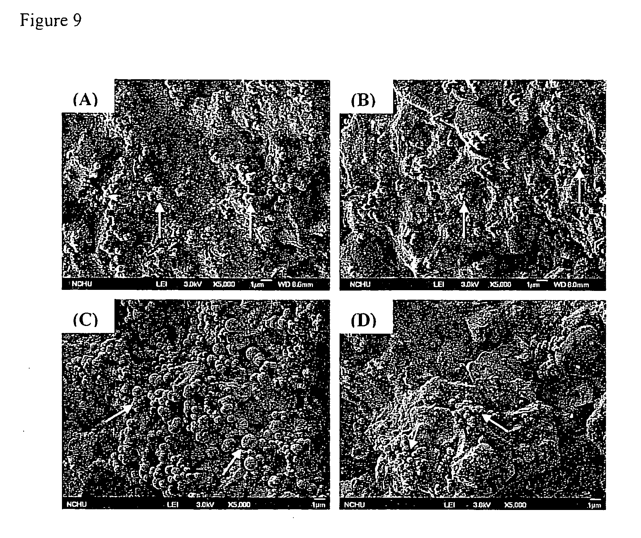

The sol-gel-derived S60C40, S50C50, S40C60, and S30C70 powders were sintered at 800°C for 2 hours. 5 wt% of gelatin (GLT) was added to the sintered powders. Then, the mixtures with and without gelatin were ball-milled using agate jars with agate grinding media in an ethanol medium for 12 hours under Retsch centrifugal ball mill S 100. After mixing with water or 5 wt% chitosan (CTS) solution, each hardened specimen that was stored in an incubator at 100% relative humidity and 37°C for one day was immersed in physiological solution for the predetermined periods of time at 37°C to evaluate the cement bioactivity. A simulated body fluid (SBF), which ionic composition is similar to that of human blood plasma, was used for immersion tests. The SBF solution consisted of 7.9949 g NaCl, 0.3528 g NaHCO₃, 0.2235 g KCI, 0.147 g K₂HPO₄, 0.305 g MgCl₂·6H₂O, 0.2775 g CaCl₂, 0.071 g Na₂SO₄ in 1000 ml distilled H₂O and was buffered to pH 7.4 with hydrochloric acid (HCl) and trishydroxymethyl aminomethane (CH₂OH)₃CNH₂). The solution in a shaker water bath was not changed daily. After immersion in SBF for different periods of time, the specimens were removed from the vials and performed the tensile testing using an EZ-Test machine. At least twelve specimens from each group were tested. DTS of almost immersed cement specimens had a greater value than the hardened cements (Fig. 7). After immersion, the specimens were removed from the vials to observe morphologies using a SEM. Figures 8 and 9 indicate that the cement specimens induce the formation of apatite spherulites, indicating the bioactivity.

### Example 9

### Cell viability and morphology

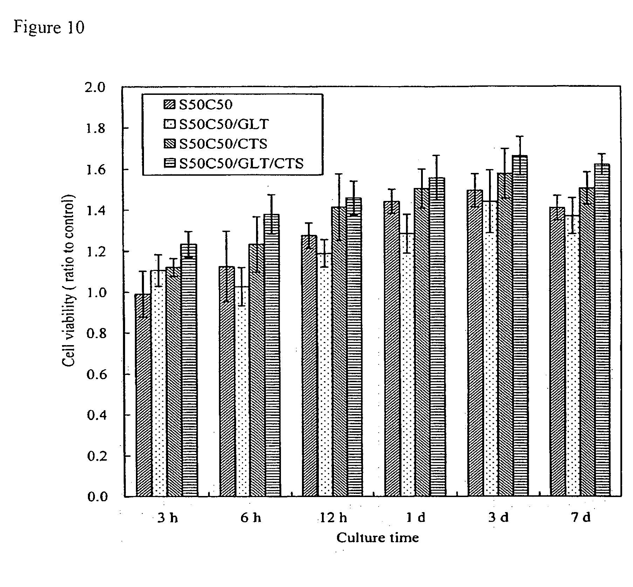

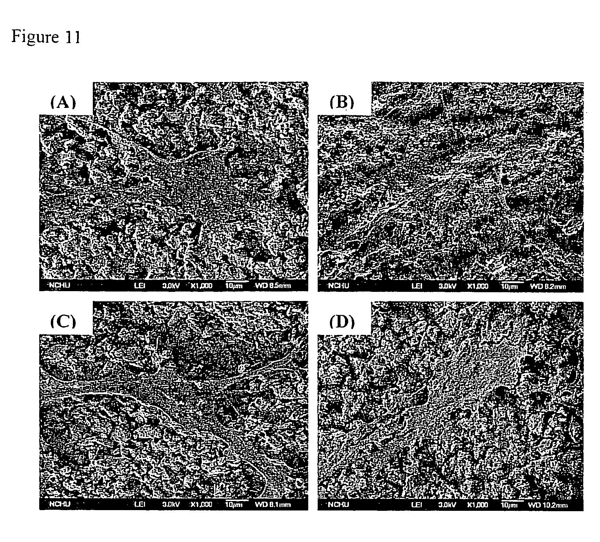

Cement biocompatibility was evaluated by incubation with human bone marrow-derived mesenchymal stem cells (hMSCs) obtained from Osiris (Worthington Biochemical, Lakewood, NJ) at passage 4. The sol-gel-derived S50C50 powders were sintered at 800°C for 2 hours. 5 wt% of gelatin (GLT) was added to the sintered powders and ground for 12 hours. After drying, in order to prepare the cement, the powder was mixed with water or 5 wt% of chitosan (CTS) solution liquid-to-powder (L/P) ratio of 0.5 mL/g. The cement specimens were stored in an incubator at 100% relative humidity and 37°C for one day. For cell culture, the 1-day setting cement discs were sterilized by soaking in 75% ethanol and exposure to ultraviolet (UV) light for 2 hour. The hMSC cells were seeded to sterilized S50C50 cement at a density of 5×10³ cells/well in a 24-well plate. The cells were grown in the osteogenic induction medium consisted of Dulbecco's modified Eagle's medium (DMEM) supplemented with 15% fetal bovine serum (FBS), 584 mg/L glutamine, 0.1 mM MEM sodium pyruvate, 100 U/mL penicillin, 100 µg/mL streptomycin, 50 mg/L ascorbic acid, 0.1 mM MEM nonessential amino acids, and 100 nM dexamethasone and 10 mM β-glycerophosphate disodium salt hydrate. Cultures were incubated at 37°C in a 5% CO₂ atmosphere for different time periods. The medium was changed every three days. The Cell Proliferation Reagent WST-1 (Roche Diagnostics, Mannheim. Germany) was used to assess cell attachment and proliferation, which is based on the cleavage of a tetrazolium salt (west-1) by the mitochondrial dehydrogenase of living cells. Briefly, three hours before the end of incubation, 100 uL of WST-1 solution (Sigma) and 900 uL of medium were added to each well. 200 µL of the solution in each well was transferred to a 96-well tissue culture plate. Plates were read in a microtiter plate reader (Bio-Rad Benchmark Plus^{™} Microplate Spectrophotometer) at 440 nm with a reference wavelength of 650 nm. Sample analysis results were obtained in triplicate from four separate experiments. hMSCs cultured on tissue culture plates (TCPs) were used as a control. In Figure 10 WST-1 assay shows that the number of viable cells increases with an increased incubation, indicating a good biocompatibility. The chitosan-containing cement specimens show a better biocompatibility compared with those specimens without chitosan. Cell morphology on the cement surfaces was examined by scanning electron microscopy (SEM). The specimens were washed by phosphate buffer solution three times and fixed with 2.5% glutaraldehyde at 4°C for 2 hours. Then the cements were dehydrated in a graded ethanol series for 20 minutes at each concentration and dried over night. The dried specimens were mounted on stubs, coated with gold layer. The cell morphology on the cement surface was observed using JEOL JSM-6700F SEM. The evaluation of SEM images confirmed that cells appeared to have firmly anchored on the cement surfaces (Fig. 11).

## Claims

1. A method for producing polymer-containing calcium silicate bone cement, comprising
(a) mixing calcium salt with silicon compound, wherein the calcium salt is selected from the group consisting of calcium oxide, calcium hydroxide, calcium acetate, calcium carbonate and calcium chloride, and wherein the silicon compound is selected from the group consisting of silicon dioxide and silicon tetraacetate;
(b) treating the mixture of step (a) with a sol-gel process;
(c) heating the mixture of step (b) ;
(d) adding gelatin to the mixture of step (c) in the range of 2-30 total wt% for enhancing plasticity;
(e) grinding the mixture of step (d) into powder; and
(f) adding the powder to water or phosphate solution.

2. The method of claim 1, wherein the mixture has a molar ratio of calcium to silicon ranging from 10 to 0.1 and more preferably a molar ratio of calcium to silicon ranging from 4 to 0.25.

3. The method of claim 1 or 2, wherein the sol-gel process comprises the following steps:
(a) mixing the mixture with a diluted solvent selected from the group consisting of nitric acid and ethanol for 1∼12 hours;
(b) placing the mixture at the temperature of 20 ∼ 100°C for 1∼7 days; and
(c) drying the mixture at -40 ∼150°C.

4. The method of any of the preceding claims, wherein the heating process comprises the following steps:
(a) heating the dried mixture to 700∼1300 °c at the rate of 1~40°C/min;
(b) maintaining the mixture at a constant temperature ranges from 700~1300 °C; and
(c) cooling the mixture to room temperature by air-cooling, water-cooling or fast cooling techniques to obtain calcium silicate powder.

5. The method of any of the preceding claims, wherein the grinding process comprises the following steps:
(a) mixing the calcium silicate powder containing the materials for enhancing plasticity with alcohol;
(b) grinding the powder with a ball bill for 0.5~ 3 days; and
(c) drying the powder at -40~60°C.

6. The method of any of the preceding claims, wherein the powder to water or phosphate solution is at a ratio of 1 g/0.3-2 mL and more preferably at a ratio of 1 g/0.4-0.8 mL.

7. The method of any of the preceding claims, wherein the phosphate solution contains an anion selected from phosphate radical (PO₄³⁻), monohydric phosphate radical (HPO₄²⁻) or dihydric phosphate radical (H₂PO₄⁻)

8. The method of any of the preceding claims, wherein the phosphate solution contains a cation selected from ammonium or a member of group I A and wherein the anion is preferably in the concentration of 0.12~5 M.

9. A composition for polymer-containing calcium silicate bone cement, comprising
(a) calcium salt selected from the group consisting of calcium oxide, calcium hydroxide, calcium acetate, calcium carbonate and calcium chloride;
(b) silicon compound selected from the group consisting of silicon dioxide and silicon tetraacetate;
(c) gelatin in the range of 2-30 total wt%; and
(d) pharmacological acceptable solution.

10. The composition of claim 9, wherein the pharmacological acceptable solution is water, sodium chloride solution, calcium chloride solution or phosphate solution.

## Patentansprüche

1. Verfahren zur Herstellung von polymerhaltigemGalciumsilikat-Knochenzement, umfassend
(a) Mischen eines Calciumsalzes mit einer Siliciumverbindung, wobei das Calciumsalz ausgewählt ist aus der Gruppe bestehend aus Calciumoxid, Calciumhydroxid, Calciumacetat, Calciumcarbonat und Calciumchlorid, und wobei die Siliciumverbindung ausgewählt ist aus der Gruppe bestehend aus Siliciumdioxid und Siliciumtetraacetat;
(b) Behandlung des Gemischs aus Schritt (a) mit einem Sol-Gel-Verfahren;
(c) Erhitzen des Gemischs aus Schritt (b);
(d) Zugabe von Gelatine zum Gemisch aus Schritt (c) im Bereich von 2-30 Gew.-%, bezogen auf das Gesamtgewicht des Gemischs,zur Erhöhung der Plastizität;
(e) Zerkleinern des Gemischs aus Schritt (d) zu Pulver; und
(f) Zugabe des Pulvers in Wasser oder Phosphatlösung.

2. Verfahren nach Anspruch 1, wobei das Gemisch ein Molverhältnis von Calcium zu Silicium im Bereich von 10 zu 0.1, besonders bevorzugt ein Molverhältnis von Calcium zu Silicium im Bereich von 4 zu 0.25, aufweist.

3. Verfahren nach Anspruch 1 oder 2, wobei das Sol-Gel-Verfahren die folgenden Schritte umfasst:
(a) Mischen des Gemischs mit einem verdünnten Lösungsmittel ausgewählt aus der Gruppe bestehend aus Salpetersäure und Ethanol für 1-12 Stunden;
(b) Aufbringen des Gemischs bei einer Temperatur von 20 - 100°C für 1 - 7 Tage; und
(c) Trocknen des Gemischs bei -40 -150°C.

4. Verfahren nach einem der vorangegangen Ansprüche, wobei der Erhitzungsvorgang die folgenden Schritte umfasst:
(a) Erhitzen des getrockneten Gemischs auf 700 - 1300°C mit einer Aufheizratevon 1 -40°C/min;
(b) Halten des Gemischs bei einer konstanten Temperatur im Bereich von 700 -1300°C; und
(c) Abkühlen des Gemischs auf Raumtemperatur mittels Luftkühlung, Wasserkühlung oder Schnellkühltechniken, um Silikatpulver zu erhalten.

5. Verfahren nach einem der vorangegangen Ansprüche, wobei der Zerkleinerungsvorgang die folgenden Schritte umfasst:
(a) Mischen des Calciumsilikatpulvers, das die Materialien zur Erhöhung der Plastizität enthält, mit Alkohol;
(b) Zerkleinern des Pulvers mit einer Kugelmühle für 0.5 - 3 Tage; und
(c) Trocknen des Pulvers bei -40 - 60°C.

6. Verfahren nach einem der vorangegangen Ansprüche, wobei das Pulver in einem Verhältnis zu Wasser oder zurPhosphatlösung von 1 g/0.3- 2mL und besonders bevorzugt in einem Verhältnis von 1g/0.4 - 0.8 mL vorliegt.

7. Verfahren nach einem der vorangegangen Ansprüche, wobei die Phosphatlösung ein Anion ausgewählt aus einem Phosphatradikal (PO₄³⁻), einem monohydrischen Phosphatradikal (HPO₄²⁻), oder einem dihydrischen Phosphatradikal (H₂PO₄⁻), enthält.

8. Verfahren nach einem der vorangegangen Ansprüche, wobei die Phosphatlösung ein Kation ausgewählt aus einem Ammonium oder einem Mitglied der Gruppe 1 A enthält, und wobei das Anionvorzugsweise In einer Konzentration von 0.12 - 5 M vorliegt.

9. Zusammensetzung fürpolymerhaltigen Calciumsilikat-Knochenzement, umfassend
(a) ein Calciumsalz ausgewählt aus der Gruppe bestehend aus Calciumoxid, Calciumhydroxid, Calciumacetat, Calciumcarbonat und Calciumchlorid;
(b) eine Siliciumverbindung ausgewählt aus der Gruppe bestehend aus Siliciumdioxid und Siliciumtetraacetat;
(c) Gelatine in einem Bereich von 2-30 Gew.-%, bezogen auf das Gesamtgewicht des Gemischs; und
(d) einepharmakologisch verträgliche Lösung.

10. Zusammensetzung nach Anspruch 9, wobei die pharmakologisch verträgliche Lösung Wasser, Natriumchlorid, Calciumchlorid oder eine Phosphatlösung ist.

## Revendications

1. Une méthode de production d'un ciment polymère d'os à base de silicate de calcium comprenant:
(a) le mixage d'un sel de calcium avec un silane, dans lequel le sel de calcium est choisi au sein du groupe composé d'oxyde de calcium, d'hydroxyde de calcium, de l'acétate de calcium, du carbonate de calcium, et du chlorure de calcium, et dans lequel le silane est choisi au sein du groupe comportant la silice, et le tetraacetate de silicium;
(b) le traitement du mélange de l'étape (a) au moyen d'un procédé sol-gel;
(c) le chauffage du mélange de l'étape (b);
(d) l'ajout de gélatine au mélange obtenu dans l'étape (c) dans une proportion de 2-30 % de poids pour l'accroissement de sa plasticité;
(e) l'abrasion du mélange de l'étape (d) pour en produire une poudre; et
(f) l'ajout de la poudre à une solution aqueuse ou phosphate.

2. La méthode de la revendication 1, dans laquelle le mélange présente un taux molaire de calcium par rapport au silicium s'étendant de 10 à 0.1., et de manière préférentielle un taux molaire de calcium par rapport au silicium s'étendant de 4 à 0.25.

3. La méthode de la revendication 1 ou 2, dans laquelle le procédé sol-gel comporte les étapes suivantes:
(a) le mixage du mélange avec un solvant dilué choisi au sein du groupe comprenant l'acide nitrique et de l'éthanol pendant 1-12 Heures.

4. La méthode selon l'une quelconque des revendications précédentes, dans laquelle le procédé de chauffage comporte les étapes suivantes:
(a) le chauffage du mélange asséché à une température de l'ordre de 700-1300 °C, à un rythme de l'ordre de 1 - 40°C/min;
(b) le maintien du mélange à une température constante s'étendant de 700 -1300 °C , et
(c) le refroidissement du mélange à la température ambiante au moyen d'un refroidissement à air, d'un refroidissement à eau, ou de techniques de refroidissement rapides en vue de l'obtention d'une poudre de silico-calcium.

5. La méthode selon l'une quelconque des revendications précédentes, dans laquelle le procédé d'abrasion comporte les étapes suivantes:
(a) le mélange de la poudre de silico-calcium contenant la substance pour améliorer la plasticité avec de l'alcool;
(b) l'abrasion de la poudre avec une roulette pendant une durée de 0.5 jour à 3 jours;
(c) séchage de la poudre à -40∼60 degrés.

6. La méthode selon l'une quelconque des revendications précédentes, dans laquelle la poudre pour la solution aqueuse ou phosphate présente un taux de 1g/0.3-2mL et préférentiellement un taux de 1g/0.4-0.8mL.

7. La méthode selon l'une quelconque des revendications précédentes, dans laquelle la solution phosphate contient un anion choisi à partir de radical phosphate (pO₄³⁻) de radical phosphate monohydrique (HPO₄²⁻) ou d'un radical phosphate dihydrique (H2PO₄⁻)

8. La méthode selon l'une quelconque des revendications précédentes, dans laquelle la solution phosphate contient un cation choisi à partir d'ammonium ou d'un membre du groupe 1 A , et dans laquelle l'anion est de préférence à une concentration de 0.12-5 M.

9. Une composition de ciment polymère d'os à base de silicate de calcium comprenant :
(a) un sel de calcium choisi au sein du group composé d'oxyde de calcium, d'hydroxyde de calcium, de l'acétate de calcium, du carbonate de calcium, et du chlorure de calcium,
(b) un silane choisi au sein du groupe comprenant la silice et le tetra-acetate de silicium;
(c) de la gélatine dans une proportion de 2-30 % de poids;
(e) d'une solution pharmacologiquement assimilable.

10. La composition selon la revendication 9, dans laquelle la solution pharmacologiquement assimilable est de l'eau, une solution de chlorure de sodium, une solution de chlorure de calcium ou une solution phosphate.
